# EUROPÄISCHE PATENTSCHRIFT

(11) **EP 0 537 488 B1**
(45) Veröffentlichungstag und Bekanntmachung des Hinweises auf die Patenterteilung: **28.05.1997**
(21) Anmeldenummer: 92115874.7
(22) Anmeldetag: 17.09.1992
(51) Int. Cl.: G01N 33/96, G01N 33/53

(54) **Künstliche Standard- und Kontrollseren zur Verwendung in Verfahren zur Bestimmung von Antikörpern**
Artificial standard and control sera to be used in assays for antibodies
Sérums de contrôle et standard artificiels pour utilisation dans des essais de determination d'anticorps

(30) Priorität: 14.10.1991 DE 4133945
(43) Veröffentlichungstag der Anmeldung: 21.04.1993
(73) Patentinhaber: BEHRINGWERKE Aktiengesellschaft, 35001 Marburg (DE)
(72) Erfinder: Brust, Stefan, W-3550 Marburg-Michelbach (DE); Friesen, Heinz-Jürgen, W-3550 Marburg 21 (DE); Nau, Günther, W-3550 Marburg-Schröck (DE); Pauly, Hans-Erwin, W-3563 Dautphetal (DE)

(56) Entgegenhaltungen:
- EP-A- 0 062 227
- EP-A- 0 291 086
- TRENDS IN BIOTECHNOLOGY Bd. 6, Nr. 2, Februar 1988, CAMBRIDGE GB Seiten 36 - 42 WILLIAMS 'Novel antibody reagents: production and potential'
- ANNALES DE BIOLOGIE CLINIQUE Bd. 48, Nr. 7, 1990, PARIS Seiten 473 - 477 HAMILTON 'Engineered human antibodies as immunologic quality control reagents'

## Beschreibung

Die Erfindung betrifft künstliche Standard- und Kontrollreagenzien für die Verwendung in immunchemischen Nachweisverfahren sowie Verfahren zur Herstellung dieser Reagenzien.

Übliche immunologische Verfahren zur Diagnose von Erkrankungen, die mit einer Bildung von spezifischen Antikörpern gegen einen Krankheitsauslöser, wie Viren, Bakterien, Allergene, Autoantigenen oder bestimmte Arzneimittel einhergehen, beruhen auf der Fähigkeit dieser Antikörper zur Komplexbildung mit antigenen Strukturen des Auslösers. Bei einigen dieser Verfahren wird eine auf den Gehalt an spezifischen Antikörpern zu prüfende Probe mit den antigenen Strukturen des Krankheitsauslösers in Kontakt gebracht, wobei diese antigenen Strukturen an geeignete Trägermaterialien befestigt sind. In der Probe enthaltene nachzuweisende, spezifische Antikörper werden als Immunkomplex an die auf dem Trägermaterial fixierten antigenen Strukturen des Krankheitsauslösers gebunden und nachgewiesen. Dazu können Antikörper bzw. andere Rezeptoren s. B. Protein A, die zur Komplexbildung mit dem spezifischen Antikörper der Probe befähigt sind, verwendet werden. Das Nachweisreagenz trägt in der Regel eine Markierung, welche die Menge des gebundenen spezifischen Antikörpers meßtechnisch erfaßbar macht. Gebräuchliche Markierungen sind: Radioaktive Isotope, Enzyme, fluoreszierende, phosphoreszierende oder lumineszierende Substanzen, Substanzen mit stabilen, ungepaarten Elektronen, Erythrozyten, Latexpartikel, Metallsole.

Für diese immunchemischen Verfahren benötigt man Kontroll- und Standardseren, welche die nachzuweisenden Antikörper in definierter Menge enthalten. Ein derartiges positives Kontrollserum erlaubt es, die Brauchbarkeit der im Test verwendeten Reagenzien zu prüfen. Darüberhinaus wird die Standardisierung und damit die Vergleichbarkeit von Testergebnissen ermöglicht, die an verschiedenen Tagen oder in verschiedenen Labors erhalten wurden. Mit Hilfe von Standardseren sind quantitative Bestimmungen möglich.

Eine häufig praktizierte Technik zur Herstellung von positiven Kontroll- oder Standardseren besteht darin, Patienten, deren Krankheit durch definierte Auslöser verursacht ist, Blut zu entnehmen, das Serum zu gewinnen und durch Mischen von Seren verschiedener Patienten das Kontroll- oder Standardserum auf einen bestimmten Gehalt an spezifischen, gegen den Krankheitsauslöser gerichtete Antikörper, einzustellen.

Die Nachteile dieser Methode liegen darin, daß eine genügende Anzahl von Personen, deren Blut diese Antikörper enthält, verfügbar sein muß. Weiterhin sprechen oft medizinische Gründe gegen eine Blutentnahme bei solchen Personen, beispielsweise bei Kindern, oder es besteht die Gefahr, daß das Blut des Erkrankten infektiös ist und geeignete Abtötungsverfahren für diesen Erreger nicht bekannt sind.

In der DE-A-31 12 334 wurden künstliche Standard- und Kontrollseren sowie Verfahren zu ihrer Herstellung und ihrer Verwendung beschrieben, die die vorstehenden Nachteile nicht aufweisen. Als Standard- und Kontrollreagenz wird ein chemisch verknüpftes Konjugat aus zwei Komponenten A und B verwendet, wobei die Komponente A im Konjugat die Fähigkeit zur Komplexbildung mit den antigenen Strukturen des Krankheitsauslösers verleiht und die Komponente B ein menschliches Immunglobulin oder Immunglobulin-Fragment derjenigen Klasse darstellt, die im immunchemischen Nachweisverfahren erfaßt werden soll. Zur Herstellung eines Konjugats gemäß der DE-A-31 12 334 muß also die Komponente B, d. h. menschliches Immunglobulin der Klasse G, M, A, D oder E in großer Menge und in höchster Reinheit aus menschlichem Serum isoliert werden. Ein identisches Vorgehen zur Herstellung von künstlichen Standard- und Kontrollreagenzien wird 1988 in der DE-A-38 00 048 beschrieben.

Die Nachteile dieser Verfahren liegen darin, daß zur Gewinnung der Immunglobuline ein aufwendiges Reinigungsverfahren erforderlich ist und diese Reinigungsprozedur zum Verlust oder zur Veränderung von antigenen Strukturen der Immunglobuline führt. So neigt beispielsweise das humane Immunglobulin M bei allen bekannten Reinigungsverfahren zur Aggregatbildung, die seine Verwendbarkeit für die Herstellung von Konjugaten der oben beschriebenen Art erschwert. Bei anderen Immunglobulinen, z. B. der Klasse IgE, liegen die üblicherweise in Humanseren vorkommenden Konzentrationen unter 100 µg/l. Für die Reinigung von humanem IgE muß deshalb auf Blutspenden von Myelom-IgE-Patienten zurückgegriffen werden, von denen weltweit nur wenige bekannt sind und deren Blutproben entsprechend selten und kostbar sind.

Kontrollseren im Sinne dieser Erfindung schließen auch Standardseren ein.

Daher bestand die Aufgabe, künstliche Standard- und Kontrollseren zu finden, die die Nachteile der oben beschriebenen Konjugate nicht aufweisen.

Überraschenderweise wurde festgestellt, daß ein geeignetes künstliches Standard- und Kontrollserum dadurch erhalten werden kann, daß ein Konjugat hergestellt wird aus einer Komponente A, die einen Bindefaktor gegen Strukturmerkmale des Krankheitsauslösers darstellt, wie z. B. einen gegen dieses Strukturmerkmal gerichteten Antikörper, und einer Komponente B, die befähigt ist zur Bindung an spezifische Strukturmerkmale des nachzuweisenden Bindefaktors gegen den Krankheitsauslöser, ohne dessen immunchemische Reaktionsfähigkeit einzuschränken. Dieses Konjugat wird in einer geeigneten Konzentration Blut, Blutbestandteilen oder Probenflüssigkeit von NichtErkrankten zugesetzt. Das resultierende Produkt wird dann zu Standard- oder Kontrollzwecken eingesetzt.

Es erwies sich, daß Konjugate aus den soeben beschriebenen Komponenten A und B reproduzierbar hergestellt werden können und in Verbindung mit dem natürlichen Gehalt an Immunglobulinen im Blut, in Blutbestandteilen oder anderen Probeflüssigkeiten von Nicht-Erkrankten ein hervorragend geeignetes Reagenz zur Funktionskontrolle bzw. zur Standardisierung erhalten werden kann.

Gegenstand der Erfindung sind somit Standard- und Kontrollseren zur Verwendung in immunchemischen Nachweisverfahren von Antikörpern bestimmter Antikörperklassen, die spezifisch gegen bestimmte Krankheitsauslöser gerichtet sind, in Körperflüssigkeiten von Säugetieren, charakterisiert dadurch, daß diese Standard- und Kontrollseren Konjugate aus einem analytspezifischen und einem antikörperspezifischen Bindungsteil in Gegenwart eines Antikörpers der spezifischen nachzuweisenden Antikörperklasse des Säugetieres enthalten, wobei das Konjugat als solches nicht als immunchemisches Äquivalent zu dem nachzuweisenden Antikörper erkannt wird.

Bevorzugt sind dabei solche Standard- oder Kontrollseren, worin die nachzuweisenden Antikörper humanen Ursprungs sind.

Ferner sind bevorzugt solche Standard-oder Kontrollseren, worin die analytspezifischen und antikörperklassenspezifischen Bindungsteile monoklonale Antikörper bzw. Antikörperfragmente sind.

Als Strukturmerkmale des Krankheitsauslösers sind Antigene, z. B. Proteine, Glykoproteine zu verstehen. Die Komponente A des Konjugates können bevorzugterweise polyklonale oder monoklonale Antikörper bzw. deren Fragmente bilden, die nach dem Fachmann bekannten Verfahren gegen Strukturmerkmale des Krankheitsauslösers hergestellt werden können, sowie Lektine oder andere Rezeptoren. Bevorzugterweise ist die Komponente A nichthumanen Ursprungs.

Nicht-human im Sinne dieser Erfindung bedeutet, daß das so bezeichnete Molekül als solches nicht von spezifischen Bindungspartnern, die zum Nachweis der Analytantikörper eingesetzt werden, erkannt wird.

Besonders bevorzugt sind monoklonale Antikörper.

Die Komponente B besteht bevorzugterweise ebenfalls aus polyklonalen oder monoklonalen Antikörpern, bzw. deren Fragmenten, die gegen den nachzuweisenden Antikörper gerichtet sind oder aus Bindefaktoren, z. B. Lektine oder Protein A, die spezifisch mit Strukturmerkmalen des nachzuweisenden Antikörpers reagieren können.

Besonders bevorzugt sind Antikörper, ganz besonders bevorzugt monoklonale Antikörper. Bevorzugterweise ist auch die Komponente B nicht-humanen Ursprungs.

Unter Konjugat im Sinne der Erfindung wird jedes Konstrukt verstanden, in dem die Komponenten A und B unter Erhaltung ihrer immunchemischen Funktion verknüpft sind.

Dem Fachmann geläufige Methoden zur Herstellung solcher Konjugate sind beispielsweise Verknüpfung durch chemische Reagenzien oder durch bioaffine Wechselwirkung. Es können jedoch auch Hybridmoleküle durch chemische Synthese, die Hybridoma-Technik oder gentechnologische Methoden erzeugt werden.

Antikörper im Sinne dieser Erfindung schließen auch die jeweils relevanten dem Fachmann an sich bekannten Antikörperfragmente ein.

Ein typisches Verfahren zur Herstellung der erfindungsgemäßen Seren sieht wie folgt aus:

Nach dem Fachmann bekannten Verfahren werden monoklonale Antikörper gegen ein Analytantigen hergestellt.

Nach dem in Beispiel 1 beschriebenen Verfahren werden die analytspezifischen Antikörper mit monoklonalen Antikörpern verknüpft, die spezifisch gegen die nachzuweisende Antikörperklasse gerichtet sind.

Das Konjugat wird z. B. durch Gelchromatographie gereinigt. Vorteilhafterweise wird das Konjugat anschließend, z. B. durch Dialyse konzentriert, bevorzugterweise auf 1 bis 10 mg/ml und nach dem Fachmann bekannten Verfahren stabilisiert. Von diesem Konjugat werden definierte Mengen zu einem bestimmten Volumen, z. B. eines normalen, analytantikörperfreien Humanserums gegeben. Dieses so erhaltene Standard-bzw. Kontrollserum kann auf dem Fachmann bekannte Weise stabilisiert und lagerfähig gemacht werden.
Zum Gebrauch wird das Serum auf dem Fachmann bekannte Weise eingesetzt.

Charakteristisch für die erfindungsgemäßen Seren ist seine universelle Anwendbarkeit. Es sind keine Einschränkungen bekannt, die eine Übertragung der in den Beispielen verwendeten Verfahren auf andere Konjugate beeinträchtigen würden.

Die erfindungsgemäßen Seren sind auch für die Bestimmung von nicht-humanen Antikörpern anwendbar, wesentlich ist, daß das eingesetzte Konjugat nicht als solches von dem in dem entsprechenden Testverfahren als Markierungsrezeptor eingesetzten Molekül erkannt wird, sonderen nur nach Bindung an ein für die jeweilige Tierspezies und Antikörperklasse spezifisches Molekül.

Die erfindungsgemäßen Standard- und Kontrollseren können Verwendung finden in einer Vielzahl von Verfahren der Human- und Veterinärdiagnostik. Als Beispiele sollen angeführt werden der Nachweis von Antikörpern verschiedener Immunglobulinklassen gegen Strukturmerkmale von Viren, z. B. Viren der Hepatitis A, B, C, verschiedener HIV-Typen, von Röteln, Cytomegalie, Masern, Mumps, Varizella, Herpes simplex und Epstein-Barr-Virus, von bakteriellen und parasitären Erregern, wie Syphilis, Borreliosis, Toxoplasmosis sowie von allergischen Erkrankungen, z. B. der Nachweis von allergiespezifischen IgE-Antikörpern, von Autoimmun-Erkrankungen sowie der Nachweis von humoralen Abwehrreaktionen im Patienten, denen möglicherweise immunogene Wirkstoffe zu diagnostischen oder therapeutischen Zwecken verabreicht wurden. Beispiele dazu sind monoklonale Antikörper gegen Tumorassoziierte Strukturen sowie rekombinante Proteine mit cytokinetischen oder Gerinnungs-Eigenschaften.

Die Erfindung wird durch die folgenden Beispiele erläutert:

### Beispiel 1

### Herstellen eines positiven Kontrollserums für Anti-HBcAg-IgM

Zu 4 mg monoklonalem Anti-HBcAg-Antikörper (in 1 ml PBS pH 7,2) werden 1 ml 0.2 M Li-BO₃/20 % Dioxan gegeben und mit einem 15 fach molaren Überschuß an N- -Maleimidobutyryloxysuccinimid (GMBS) versetzt und 1 h bei Raumtemperatur inkubiert. Das nicht umgesetzte heterobifunktionelle Reagenz wird über Gelchromatographie (Sephadex® G-25) mit 0,1 molarem Natriumphosphatpuffer + 5 mM Nitrilotriessigsäure (NTA) pH 6,0 abgetrennt.

2 mg monoklonaler Anti-Human-IgM-Antikörper (in 2 ml 10 mM Natriumphosphat, 100 mM NaCl, pH 7,4) wird mit einem 24fach molarem Überschuß an N-Succinimidyl-3-(2-pyridyldithio)propionat (SPDP) 30 Minuten bei Raumtemperatur inkubiert, danach mit 100fach molarem Überschuß gegenüber SPDP mit Dithiothreitol (DTT) 15 Minuten bei RT reduziert. Nach erfolgter Reduktion wird der Überschuß an DTT und SPDP über Gelchromatographie (Sephadex® G-25) mit 0.1 M Natriumphosphat/5 mM NTA pH 6,0 entfernt.

Das SH-aktivierte Anti-human IgM wird mit dem aktivierten Anti-HBcAg 2 Stunden bei RT inkubiert und anschließend mit 1/10 Vol 0,1 M N-Ethylmaleimid abgestoppt.
Das Konjugat wurde durch Gelchromatographie (ACA 34, LKB) mit 50 mM TRIS/HCl pH 7,4 gereinigt und anschließend auf 3 ml ankonzentriert und mit HSA stabilisiert. Von diesem Konjugat (Konz. = 2 mg/ml) werden 50 µl, 25 µl, 12,5 µl und 6,25 µl 1 ml in normales Humanserum (Anti-HBc-IgM negativ) pipettiert, über 0,2 µm Sartorius Membranfilter filtriert und bei +2 bis +8°C gelagert.

### Beispiel 2

### Anwendung der Konjugate im Enzymimmunoassay

In einem Enzymimmunoassay zum Nachweis von IgM-Antikörpern gegen HBcAg (Enzygnost^{R} Anti-HBcAg/IgM-Testkit der Behringwerke AG, Marburg, FRG) werden 10 µl eines 1:100 vorverdünnten Serums bzw. der gemäß Beispiel 1 hergestellten künstlichen positiven Kontrolle und 90 µl Probenpuffer in mit Anti-Human-IgM beschichteten Mikrotitrationsplatten eine Stunde bei 37°C inkubiert, gemäß Packungsbeilage gewaschen, eine Stunde bei 37°C mit 100 µl HBcAg-POD-Konjugat inkubiert, erneut gewaschen, mit 100 µl Tetramethylbenzidin(TMB)-Substratlösung gemäß Packungsbeilage 30 Min. bei Raumtemperatur inkubiert, mit 100 µl 0,5 M H₂SO₄-Lösung abgestoppt und bei 450 nm photometriert.
Das Markerenzym Peroxidase katalysiert die Umsetzung des Chromogens TMB zum Farbstoff; die nach 30 Min. erzeugte Farbe ist proportional zum Gehalt der Probe an gegen HBcAg gerichteten Antikörpern. Die für die in Beispiel 1 beschriebene Verdünnungsreihe der künstlichen positiven Kontrolle erhaltenen Extinktionswerte sind im Vergleich zur Extinktion der negativen Kontrolle in Tabelle 1 dargestellt:

**Tabelle 1:**

| Verdünnung der künstl. pos. Kontrolle | Extinktionswerte bei 450 nm | Extinktionswerte der neg. Kontrolle bei 450 nm |
|---|---|---|
| 1 : 20 | > 3.000 | |
| 1 : 40 | 2.583 | 0.056 |
| 1 : 80 | 1.385 | |
| 1 : 160 | 0.638 | |

### Beispiel 3

### Herstellen eines Konjugates aus Anti-Human-IgM und eines Anti-Herpes-simplex-Virus (HSV)-F(ab')-Fragments

10 mg monoklonaler Antikörper gegen HSV (in 2 ml 50 mM Natriumacetat Puffer pH 4.3) werden mit 1 mg Pepsin 24 h bei 37° C inkubiert, das Reaktionsgemisch mit ca. 0,2 ml 2N NaOH auf pH 7.2 gestellt. Das F(ab')₂-Fragment wird über Gelchromatographie (ACA-44 Fa. LKB) mit 50 mM TRIS/HCl pH 7.4 gereinigt und anschließend auf 1 ml ankonzentriert (ca. 5 mg F(ab')₂-Fragment) und mit 0,1 ml 0,1 M Cysteamin HCl-Lösung 60 Minuten bei RT zu F(ab') reduziert und anschließend über Sephadex® G-25 (Säule 1 cm Vol. 10 ml) chromatographiert. Das reduzierte Anti-HSV-F(ab')-Fragment wird mit 5 mg aktiviertem Anti-Human-IgM-maleimid (siehe oben) 1 Stunde bei 37°C umgesetzt. Nicht umgesetzter Antikörper F(ab')-Fragment wird mittels Gelchromatographie (ACA-34 LKB) vom eigentlichen Konjugat mit 50 mM TRIS/HCl pH 7.4 abgetrennt, anschließend auf 5 ml ankonzentriert und mit HSA stabilisiert.

### Beispiel 4

### Anti GM-CSF-IgM pos. Kontrollseren

Analog zu Beispiel 3 wurde ein F(ab')-Fragment eines Anti-GM-CSF-Antikörpers vom Kaninchen mit einem Anti-Human-IgM-Antikörper von der Maus konjugiert.

## Patentansprüche

1. Standard- und Kontrollseren zur Verwendung in immunchemischen Nachweisverfahren von Antikörpern bestimmter Antikörperklassen, die spezifisch gegen bestimmte Krankheitsauslöser gerichtet sind, in Körperflüssigkeiten von Säugetieren, dadurch gekennzeichnet, daß sie Konjugate aus einem analytspezifischen und einem antikörperspezifischen Bindungsteil in Gegenwart eines Antikörpers der spezifischen nachzuweisenden Antikörperklasse des Säugetieres enthalten, wobei das Konjugat als solches nicht als immunchemisches Äquivalent zu dem nachzuweisenden Antikörper erkannt wird.

2. Standard- oder Kontrollserum gemäß Anspruch 1, dadurch gekennzeichnet, daß die nachzuweisenden Antikörper humanen Ursprungs sind.

3. Standard- oder Kontrollserum gemäß Anspruch 1, dadurch gekennzeichnet, daß der analytspezifische und antikörperklassenspezifische Bindungsteil monoklonale Antikörper bzw. Antikörperfragmente sind.

## Revendications

1. Sérums de contrôle et de référence pour utilisation dans des procédés immunochimiques de détection, dans des liquides corporels de mammifères, d'anticorps appartenant à des classes déterminées d'anticorps, qui sont spécifiquement dirigés contre des agents pathogènes déterminés, caractérisés en ce qu'ils contiennent des conjugués constitués d'un segment de liaison spécifique d'analyte et d'un segment de liaison spécifique d'anticorps, en présence d'un anticorps de la classe d'anticorps spécifique à détecter du mammifère, le conjugué en tant que tel n'étant pas reconnu comme équivalent immunochimique de l'anticorps à détecter.

2. Sérum de contrôle et de référence selon la revendication 1, caractérisé en ce que les anticorps à détecter sont d'origine humaine.

3. Sérum de contrôle et de référence selon la revendication 1, caractérisé en ce que le segment de liaison spécifique d'analyte et le segment de liaison spécifique de la classe d'anticorps sont des anticorps ou fragments d'anticorps monoclonaux.

## Claims

1. Standard and control sera for use in immunochemical detection methods for antibodies of particular antibody classes, which are directed specifically against particular pathogens, in body fluids from mammals, which contain conjugates of an analyte-specific and of an antibody-specific binding portion in the presence of an antibody of the specific antibody class which is to be detected in the mammal, where the conjugate as such is not recognized as immunochemical equivalent to the antibody to be detected.

2. A standard or control serum as claimed in claim 1, wherein the antibodies to be detected are of human origin.

3. A standard or control serum as claimed in claim 1, wherein the analyte-specific and antibody class-specific binding portions are monoclonal antibodies or antibody fragments.
